(19) Europäisches Patentamt
European Patent Office
Office européen des brevets

(11) **EP 4 331 722 A1**

(12) **EUROPEAN PATENT APPLICATION**
published in accordance with Art. 153(4) EPC

(43) Date of publication:
06.03.2024 Bulletin 2024/10

(21) Application number: 21939224.8

(22) Date of filing: 27.04.2021

(51) International Patent Classification (IPC):
*B01J 23/745* (2006.01)    *C07C 29/156* (2006.01)
*C07C 31/08* (2006.01)

(52) Cooperative Patent Classification (CPC):
**B01J 23/745; C07C 29/156; C07C 31/08;**
Y02P 20/52

(86) International application number:
**PCT/JP2021/016831**

(87) International publication number:
**WO 2022/230063 (03.11.2022 Gazette 2022/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Toyo Tire Corporation
Itami-shi, Hyogo 664-0847 (JP)**

(72) Inventors:
• **NAKAMURA, Norihiko**
  **Itami-shi, Hyogo 664-0847 (JP)**
• **TSUBAKI, Noritatsu**
  **Toyama-shi, Toyama 930-8555 (JP)**
• **YANG, Guohui**
  **Toyama-shi, Toyama 930-8555 (JP)**

(74) Representative: **Ricker, Mathias
Wallinger Ricker Schlotter Tostmann
Patent- und Rechtsanwälte Partnerschaft mbB
Zweibrückenstraße 5-7
80331 München (DE)**

(54) **ETHANOL SYNTHESIS CATALYST, AND METHOD FOR PRODUCING ETHANOL**

(57) The ethanol synthesis catalyst is a catalyst for synthesizing ethanol from carbon dioxide, and includes at least one carrier selected from the group consisting of $ZrO_2$, $Al_2O_3$, and $TiO_2$; Fe and Cu supported on the carrier; and Na and/or K supported on the carrier. In one embodiment, the catalyst including iron oxide and copper oxide as the Fe and Cu supported on the carrier can be used, hydrogen can be supplied to the catalyst to reduce iron oxide and copper oxide, and a reaction gas containing $CO_2$ can be supplied to the catalyst after reduction to obtain ethanol from $CO_2$.

FIG. 1

**Description**

Technical Field

An embodiment of the present invention relates to a catalyst for synthesizing ethanol from carbon dioxide and a production method of ethanol in which the catalyst is used.

Background Art

Ethanol is widely used in chemicals (chemical solvents and the like), housewares (pharmaceuticals, cosmetics, detergents and the like), foods and beverages (miso, soy sauce, processed foods, food preservatives and the like) and fuels, and is essential for people's lives and industrial activities. Ethanol is currently produced by a direct hydration process of ethylene or a fermentation method in which biomass is used as a raw material. However, due to global warming and competition between resource use and food use of biomass, a new synthesis route is required. Thus, a synthesis method of recycling carbon dioxide ($CO_2$), which is a causative agent of global warming and is emitted into the atmosphere in large amounts every year, as ethanol is attracting attention.

For example, Non Patent Literature 1 discloses synthesis of ethanol from $CO_2$ using a catalyst in which Rh, Fe, and Li have been supported on $TiO_2$ nanoparticles ($TiO_2NPs$) by impregnation method. Specifically, a $CO_2$ conversion of 15% and an ethanol selectivity of 32% are achieved under reaction conditions of 250°C and 3.0 MPa by 2.5% by mass $RhFeLi/TiO_2NPs$ (Rh : Fe : Li (molar ratio) = 1 : 1 : 1).

Non Patent Literature 2 discloses synthesis of an alcohol having 2 or more carbon atoms from $CO_2$ using a catalyst prepared by a coprecipitation method in which Cs has been supported on CuFeZn. Specifically, a $CO_2$ conversion of 36.6% and selectivity of alcohol having 2 or more carbon atoms of 19.8% are achieved under reaction conditions of 330°C and 5.0 MPa by 3% by mass $Cs/Cu_{0.8}Fe_1Zn_1$ (Cu : Fe : Zn (molar ratio) = 0.8 : 1 : 1).

Citation List

Non Patent Literature

NPL 1: Chengsheng Yang, Rentao Mu, Guishuo Wang, Jimin Song, Hao Tian, Zhi-Jian Zhao, Jinlong Gong, Chemical Science, 10, 3161-3167 (2019)
NPL 2: Di Xu, Mingyue Ding, Xinlin Hong, Guoliang Liu, Shik Chi Edman Tsang, ACS Catalysis, 10, 5250-5260 (2020)

Summary of Invention

Technical Problem

Direct synthesis of ethanol from $CO_2$ is a promising route, but it is difficult to achieve high $CO_2$ conversion and high ethanol selectivity simultaneously. Thus, it is presumed that the development of a catalyst that has a high $CO_2$ conversion in the direct synthesis method from $CO_2$ and highly selectively synthesizes ethanol is an important need in the supply of many chemicals, housewares, beverages, and fuels.

An embodiment of the invention aims to provide an ethanol synthesis catalyst capable of efficiently synthesizing ethanol from $CO_2$ and a production method of ethanol using the same.

Solution to Problem

The ethanol synthesis catalyst according to an embodiment of the invention is a catalyst for synthesizing ethanol from carbon dioxide, including: at least one carrier selected from the group consisting of $ZrO_2$, $Al_2O_3$, and $TiO_2$; Fe and Cu supported on the carrier; and Na and/or K supported on the carrier.

The ethanol synthesis catalyst according to one embodiment can include iron oxide and copper oxide as the Fe and Cu supported on the carrier.

The ethanol synthesis catalyst according to one embodiment can include at least one selected from the group consisting of $Fe^0$ and $Fe_3O_4$ as the Fe supported on the carrier, and include $Cu^0$ as the Cu supported on the carrier.

The production method of ethanol according to an embodiment of the invention includes obtaining ethanol from carbon dioxide in the presence of a catalyst including at least one carrier selected from the group consisting of $ZrO_2$, $Al_2O_3$, and $TiO_2$; Fe and Cu supported on the carrier; and Na and/or K supported on the carrier.

In the production method of ethanol according to one embodiment, the catalyst including iron oxide and copper oxide as the Fe and Cu supported on the carrier can be used, hydrogen can be supplied to the catalyst to reduce iron oxide and copper oxide, and a reaction gas containing carbon dioxide can be supplied to the catalyst after reduction to obtain ethanol from carbon dioxide. In that case, the catalyst can include $Fe_2O_3$ in a state before reduction, and include at least one selected from the group consisting of $Fe^0$ and $Fe_3O_4$ in a reduced state as the Fe supported on the carrier. The catalyst can also include CuO in a state before reduction and include $Cu^0$ in a reduced state as the Cu supported on the carrier.

In the ethanol synthesis catalyst and the production method of ethanol according to the embodiment, the catalyst can include at least one selected from the group consisting of $Na_2CO_3$, $K_2CO_3$, NaCl, KCl, NaOH, KOH, $NaNO_3$, $KNO_3$, $Na_2SO_4$, and $K_2SO_4$ as the Na and/or K supported on the carrier.

In the ethanol synthesis catalyst and the production method of ethanol according to the embodiment, the content of Fe and Cu based on the whole of the catalyst can be 5 to 35% by mass. Amolar ratio Fe/Cu between Fe and Cu can be 0.1 to 2.5. The content of Na and/or K based on the whole of the catalyst can be 0.1 to 10% by mass.

Advantageous Effects of Invention

According to the embodiment of the invention, ethanol can be efficiently synthesized from $CO_2$.

Brief Description of Drawings

[FIG. 1] FIG. 1 is a graph showing XRD analysis results of catalysts of Examples 1 to 4;
[Fig. 2] FIG. 2 is a graph of adsorption isotherms of catalysts of Examples 1 to 4.
[FIG. 3] FIG. 3 is a graph showing $H_2$-TPR results of catalysts of Examples 1 to 4.
[FIG. 4] FIG. 4 is a conceptual diagram of the reactor used in Examples.

Description of Embodiments

Hereinafter, the ethanol synthesis catalyst (hereinafter may be simply referred to as the catalyst) according to the present embodiment and the production method of ethanol using the same will be described.

[Ethanol synthesis catalyst]

The catalyst according to the embodiment is a catalyst for synthesizing ethanol from $CO_2$, and at least one inorganic powder selected from the group consisting of $ZrO_2$ (zirconium oxide), $Al_2O_3$ (aluminum oxide), and $TiO_2$ (titanium oxide) is used as the carrier. $ZrO_2$, $Al_2O_3$, and $TiO_2$ are presumed to have activity in reverse water gas shift reaction (RWGS) represented by the following formula and promote $CO_2$ conversion.

$$CO_2 + H_2 \rightarrow CO + H_2O$$

As a catalyst carrier, among those described above, at least one selected from the group consisting of $ZrO_2$ and $Al_2O_3$ is preferably used, and $ZrO_2$ is more preferably used.

As the metal to be supported on the catalyst carrier, iron (Fe) and copper (Cu) are used. It is presumed that by Fe and Cu being supported on $ZrO_2$, $Al_2O_3$, or $TiO_2$, RWGS can be promoted and the synthesis of ethanol from CO (carbon monoxide) produced by RWGS can be promoted.

In one embodiment, the catalyst can include iron oxide and copper oxide as the Fe and Cu supported on the carrier. That is, Fe and Cu can be supported on the carrier as iron oxide and copper oxide, respectively. Examples of the iron oxide include at least one selected from the group consisting of $Fe_2O_3$ (iron (III) oxide), $Fe_3O_4$ (triiron tetraoxide), and FeO (iron (II) oxide). Examples of the copper oxide include at least one selected from the group consisting of CuO (copper (II) oxide) and $Cu_2O$ (I). Fe and Cu are preferably supported on the carrier as $Fe_2O_3$ and CuO in a state before reduction of the catalyst.

In one embodiment, it is preferable that the catalyst include at least one selected from the group consisting of $Fe^0$ and $Fe_3O_4$ as the Fe supported on the carrier, and include $Cu^0$ as the Cu supported on the carrier. Specifically, it is preferable that the catalyst include $Fe_2O_3$ in a state before reduction, include at least one selected from the group consisting of $Fe^0$ and $Fe_3O_4$ in a reduced state as the Fe supported on the carrier, and include CuO in a state before reduction, and include $Cu^0$ in a reduced state as the Cu supported on the carrier. $Fe^0$ and $Cu^0$ represent the metallic state of oxidation number 0.

Thus, it is preferable that Fe and Cu be supported on the carrier as $Fe_2O_3$ and CuO in a state before reduction of the catalyst, and be supported on the carrier as $Fe^0$ and/or $Fe_3O_4$, and $Cu^0$ in a reduced state. The catalyst more preferably includes $Fe^0$, $Fe_3O_4$, and $Cu^0$ in a reduced state.

Ethanol can be synthesized by supplying $CO_2$ to the catalyst in a reduced state. Specifically, $Fe_3O_4$ is presumed to have activity in RWGS. $Fe_3O_4$ is also presumed to become iron carbide ($Fe_xC_y$, for example, $Fe_5C_2$) through a reduction reaction with CO produced by RWGS. $Fe^0$, iron carbide, and $Cu^0$ are presumed to have activity in ethanol synthesis from CO produced by RWGS.

The content of Fe and Cu in the catalyst is not particularly limited, but is preferably 5% by mass or more and preferably 35% by mass or less. In the specification, the content of Fe and Cu means the percentage of the mass (total mass of iron element and copper element) of Fe and Cu included in the catalyst as elements when the mass of the whole of the catalyst is 100% by mass. The content of Fe and Cu can be measured, for example, by X-ray fluorescence spectroscopy (XRF). The content of Fe and Cu is more preferably 10% by mass or more, and more preferably 30% by mass or less.

The molar ratio Fe/Cu between Fe and Cu is not particularly limited, but is preferably 0.1 or more, and preferably 2.5 or less. In the specification, the molar ratio Fe/Cu is the molar ratio of the iron element to the copper element. The molar ratio Fe/Cu is more preferably 0.2 or more, further preferably 0.3 or more, and further preferably 0.4 or more. The molar ratio Fe/Cu is more preferably 2.0 or less, and further preferably 1.5 or less.

Na (sodium) and/or K (potassium) are further supported on the carrier. That is, the catalyst includes at least one alkali metal selected from the group consisting of Na and K. As the alkali metal, Na is particularly preferable. It is presumed that by these alkali metals being supported, the production of iron carbide and the adsorption of $CO_2$ are promoted. According to the embodiment, by Fe and Cu being supported, and Na and/or K further being supported on $ZrO_2$, $Al_2O_3$ and/or $TiO_2$, the $CO_2$ conversion and the ethanol selectivity can be improved, and ethanol can be efficiently synthesized.

In one embodiment, Na and/or K can be supported on the carrier as $Na^+$ and/or $K^+$. More specifically, the catalyst can include at least one selected from the group consisting of $Na_2CO_3$, $K_2CO_3$, NaCl, KCl, NaOH, KOH, $NaNO_3$, $KNO_3$, $Na_2SO_4$, and $K_2SO_4$ as the Na and/or K supported on the carrier. That is, Na and/or K can be supported on the carrier as these sodium compounds and potassium compounds. Among these, they are preferably supported as carbonates, that is, $Na_2CO_3$ and/or $K_2CO_3$, because they are less likely to adversely affect the ethanol synthesis reaction.

The content of Na and/or K in the catalyst is not particularly limited, but is preferably 0.1% by mass or more and preferably 10% by mass or less. In the specification, the content of Na and/or K means the percentage of the mass (total mass of sodium element and potassium element) of Na and/or K included in the catalyst as elements when the mass of the whole of the catalyst is 100% by mass. The content of Na and/or K can be measured, for example, by X-ray fluorescence spectroscopy (XRF). The content of Na and/or K is preferably 0.5% by mass or more, more preferably 1% by mass or more, and preferably 5% by mass or less, more preferably 3% by mass or less.

The BET specific surface area of the catalyst according to the embodiment is not particularly limited, and can be, for example, 100 to 1000 $m^2$/g, 200 to 700 $m^2$/g, or 250 to 500 m2/g.

The average pore diameter of the catalyst according to the embodiment is not particularly limited, and can be, for example, 0.5 to 50 nm, 1 to 30 nm, or 2 to 10 nm.

The pore volume of the catalyst according to the embodiment is not particularly limited, and can be, for example, 0.2 to 3.0 $cm^3$/g, 0.3 to 2.5 $cm^3$/g, or 0.5 to 2.0 $cm^3$/g.

The production method of the catalyst according to the embodiment is not particularly limited, but polymerized complex method is preferably used to highly disperse Fe and Cu. In one embodiment, it is preferable that Fe and Cu be supported on the carrier by polymerized complex method and melt impregnation method, and Na and/or K be supported on the obtained FeCu-supported carrier by impregnation method (preferably IW (incipient Wetness) method) to prepare the catalyst.

Specifically, an oxycarboxylic acid such as citric acid, metal salts of Fe and Cu, and a glycol such as ethylene glycol or propylene glycol are uniformly dissolved in water to form a metal oxycarboxylic acid complex. The carrier is added to this aqueous solution and dispersed uniformly. Then, the obtained dispersion is heated to perform a dehydration esterification reaction between the carboxy group of the oxycarboxylic acid and the hydroxy group of the glycol. The polymer gel thus obtained is dried and calcined to obtain a carrier (FeCu-supported carrier) in which Fe and Cu are supported. After calcination, the carrier can be pulverized and sized, as needed.

Examples of metal salts of Fe include, for example, iron (III) nitrate, iron (II) acetate, and hydrates thereof. Examples of metal salts of Cu include, for example, copper (II) nitrate, copper (II) acetate, and hydrates thereof. The calcination temperature is not particularly limited, and can be, for example, 300 to 700°C.

Then, the FeCu-supported carrier is impregnated little by little with an aqueous solution of an Na salt and/or a K salt by dropping or the like. Then, the carrier is vacuum dried to obtain the catalyst according to the embodiment in which Na and/or K are supported on the FeCu-supported carrier. After vacuum drying, the carrier can be pulverized and further subjected to shape forming such as sizing, as needed.

Examples of the Na salt and/or the K salt include, for example, $Na_2CO_3$, $K_2CO_3$, NaCl, KCl, NaOH, KOH, $NaNO_3$, $KNO_3$, $Na_2SO_4$, and $K_2SO_4$, and any one of these can be used, or two or more of these can be used in combination.

[Production method of ethanol]

The production method of ethanol according to the embodiment includes obtaining ethanol from $CO_2$ in the presence of the catalyst according to the embodiment described above. For this, $CO_2$ should be brought into contact with the catalyst.

Specifically, the catalyst is capable of synthesizing ethanol from $CO_2$ in a reduced state. Thus, it is preferable that the catalyst including iron oxide and copper oxide as the Fe and Cu supported on the carrier be used, hydrogen be supplied to the catalyst to reduce the iron oxide and copper oxide, and a reaction gas containing $CO_2$ be supplied to the catalyst in a reduced state. Thereby ethanol can be synthesized from $CO_2$. It is preferable that the catalyst include $Fe_2O_3$ in a state before reduction, include at least one selected from the group consisting of $Fe^0$ and $Fe_3O_4$ in a reduced state as the Fe supported on the carrier, and include CuO in a state before reduction, and include $Cu^0$ in a reduced state as the Cu supported on the carrier.

The reduction of the catalyst and the synthesis reaction of ethanol are preferably performed in a gas phase. Reduction of the catalyst can be performed by passing a reducing gas containing hydrogen through a catalyst bed containing the catalyst. The synthesis reaction of ethanol can be performed by passing a reaction gas containing $CO_2$ through the catalyst bed after reduction.

The reducing conditions of the catalyst are not particularly limited, and the temperature of the catalyst bed can be, for example, 200 to 500°C, or 300 to 400°C. The pressure can be, for example, 0.1 to 1.0 MPa, or 0.1 to 0.2 MPa. The reducing gas can be composed of, for example, only hydrogen gas, or can contain an inert gas such as nitrogen or argon together with hydrogen gas.

The conditions of synthesis reaction of ethanol are not particularly limited, and the temperature of the catalyst bed can be, for example, 150 to 400°C, or 250 to 350°C. The reaction pressure can be, for example, 1.0 to 10.0 MPa, or 3.0 to 5.0 MPa.

The reaction gas preferably contains hydrogen and $CO_2$, and can contain an inert gas such as nitrogen or argon. The volume ratio ($H_2/CO_2$) of hydrogen to $CO_2$ is not particularly limited, but is preferably 1.0 to 5.0, and more preferably 2.5 to 3.5.

The reaction type can be a continuous flow type or a batch type. The reaction form is not particularly limited, and can be any of a fixed bed, a moving bed, and a fluidized bed. The type of the reactor is also not particularly limited, and for example, a tube reactor or the like can be used.

The product obtained after the reaction can be purified by distillation or the like, as needed. Thereby by-products such as alkanes such as methane, ethane, propane and butane and alcohols other than ethanol such as methanol and propanol can be removed.

Examples

Hereinafter, Examples are shown, but the invention is not limited to these Examples.

[Measuring Method of Supported Amounts of Fe and Cu and Molar Ratio between Fe and Cu]

Using a wavelength dispersive X-ray fluorescence spectrometer (XRF; PW2404R, manufactured by Spectris Co., Ltd.), the content of Fe and Cu and the molar ratio of Fe/Cu of the catalyst before reduction were measured. The catalyst was powdered and measured under a He atmosphere.

[Measuring Method of BET Specific Surface Area]

Using a BET specific surface area measuring apparatus (NOVA2200e, manufactured by Quantachrome), the BET specific surface area of the catalyst before reduction was measured. Into a 9 mL cell, 0.02 to 0.05 g of the powdered catalyst was placed and pretreated at 200°C for 2 hours under vacuum. Then, measurement was performed by 79-point method using nitrogen as an adsorption gas.

[Measuring Method of pore volume and average pore diameter]

In the same manner as in the measurement of the BET specific surface area, using a BET specific surface area measuring apparatus (NOVA2200e, manufactured by Quantachrome), the pore volume and the average pore diameter of the catalyst before reduction were measured. Into a 9 mL cell, 0.02 to 0.05 g of the powdered catalyst was placed and pretreated at 200°C for 2 hours under vacuum. Then, measurement was performed by 79-point method using nitrogen as an adsorption gas.

[Measuring Method of Particle Size of Cu]

Using an X-ray diffractometer (XRD; Ultima IV-NS, manufactured by Rigaku Corporation), the crystal size of Cu of the catalyst after the reaction was measured. Using CuK$\alpha$ rays, measurement was performed under the conditions of 40 kV, 20 mA, and $\theta$ = 3° to 80°. Then, the crystal size of Cu was calculated using the half-value width of the peak near $\theta$ = 43°.

[Example 1: Preparation of Na-FeCu/ZrO$_2$ catalyst]

20% FeCu/ZrO$_2$ in which Fe$_2$O$_3$ and CuO (Fe/Cu = 1/1; molar ratio) are supported on ZrO$_2$ so that an supported amount as Fe$_2$O$_3$ and CuO is 20% by mass was prepared by polymerized complex method and melt impregnation method. Specifically, 1.21 g of Cu(NO$_3$)$_2$·3H$_2$O (99.0% by mass, manufactured by FUJIFILM Wako Pure Chemical Corporation), 2.02 g of Fe(NO$_3$)$_3$·9H$_2$O (99.0% by mass, manufactured by FUJIFILM Wako Pure Chemical Corporation), 2.30 g of citric acid (99.0% by mass, manufactured by FUJIFILM Wako Pure Chemical Corporation), and 0.15 g of ethylene glycol (99.0% by mass, manufactured by Sigma-Aldrich) were dissolved in 40 mL of ion-exchanged water until uniform. To the resulting aqueous solution, 3.19 g of ZrO$_2$ (98.0% by mass, manufactured by FUJIFILM Wako Pure Chemical Corporation) was added, and the mixture was left to stand overnight with stirring. Then, the resulting product was evaporated to dryness at 120°C with stirring. After drying, the temperature was raised from room temperature (30°C) to 400°C at a raising rate of 3°C/min, and the product was calcined at 400°C for 2 hours to remove the polymer of citric acid and ethylene glycol. Then, the temperature was continuously raised from 400°C to 650°C at a raising rate of 5°C/min, and the product was calcined at 650°C for 5 hours. Thereby, 20% FeCu/ZrO$_2$ was obtained.

Then, Na$_2$CO$_3$ was supported on 20% FeCu/ZrO$_2$ by IW method so that the content of Na based on the whole of the catalyst would be 2% by mass. Specifically, 0.047 g of Na$_2$CO$_3$ (99.8% by mass, manufactured by FUJIFILM Wako Pure Chemical Corporation) was dissolved in 2.0 g of ion-exchanged water, and the aqueous solution was dropped little by little at room temperature to 1.0 g of 20% FeCu/ZrO$_2$. Then the resulting product was dried under vacuum at 60°C overnight. Through the above operations, an Na-FeCu/ZrO$_2$ catalyst (Fe : Cu (molar ratio) = 1 : 1, content of Na = 2.0% by mass) was obtained.

[Examples 2 to 4: Preparation of Na-Fe$_m$Cu$_n$/ZrO$_2$ catalyst]

Catalysts of Examples 2 to 4 were prepared in the same manner as in Example 1 except that the supported amount as Fe$_2$O$_3$ and CuO of 20% by mass was not changed, and the Fe/Cu molar ratios of the nitrates of Fe and Cu charged in polymerized complex method were changed to 2/1, 1/2, and 1/3. The catalyst of Example 2 is an Na-Fe$_2$Cu/ZrO$_2$ (Fe/Cu = 2/1; molar ratio) catalyst. The catalyst of Example 3 is an Na-FeCu$_2$/ZrO$_2$ (Fe/Cu = 1/2; molar ratio) catalyst. The catalyst of Example 4 is an Na-FeCu$_3$/ZrO$_2$ (Fe/Cu = 1/3; molar ratio) catalyst.

[Comparative Example 1: Preparation of Na-Fe catalyst]

The temperature of Fe(NO$_3$)$_3$·9H$_2$O (99.0% by mass, manufactured by FUJIFILM Wako Pure Chemical Corporation) was raised to 400°C at a raising rate of 3°C/min, and the Fe(NO$_3$)$_3$·9H$_2$O was calcined at 400°C for 5 hours. Thereby, an Fe carrier was obtained.

Then, by IW method, Na$_2$CO$_3$ was supported on the Fe carrier so that the content of Na based on the whole of the catalyst would be 2% by mass. Specifically, 0.047 g of Na$_2$CO$_3$ (99.8% by mass, manufactured by FUJIFILM Wako Pure Chemical Corporation) was dissolved in 1.0 g of ion-exchanged water, and the aqueous solution was dropped little by little at room temperature to 1.0 g of the Fe carrier. Then the resulting product was dried under vacuum at 60°C overnight. Through the above operations, an Na-Fe catalyst (content of Na = 2.0% by mass) was obtained.

[Comparative Example 2: Preparation of Na-FeCu catalyst]

An FeCu carrier was prepared by polymerized complex method. Specifically, 1.21 g of Cu(NO$_3$)$_2$·3H$_2$O (99.0% by mass, manufactured by FUJIFILM Wako Pure Chemical Corporation), 2.02 g of Fe(NO$_3$)$_3$·9H$_2$O (99.0% by mass, manufactured by FUJIFILM Wako Pure Chemical Corporation), 2.30 g of citric acid (99.0% by mass, manufactured by FUJIFILM Wako Pure Chemical Corporation), and 0.15 g of ethylene glycol (99.0% by mass, manufactured by Sigma-Aldrich) were dissolved in 40 mL of ion-exchanged water until uniform. Then, the resulting product was evaporated to dryness at 120°C with stirring. After drying, the temperature was raised from room temperature (30°C) to 400°C at a raising rate of 3°C/min, and the product was calcined at 400°C for 2 hours to remove the polymer of citric acid and ethylene glycol. Then, the temperature was continuously raised from 400°C to 650°C at a raising rate of 5°C/min, and the product was calcined at 650°C for 5 hours. Thereby, an FeCu carrier was obtained.

Then, by IW method, $Na_2CO_3$ was supported on the FeCu carrier so that the content of Na based on the whole of the catalyst would be 2% by mass. Specifically, 0.047 g of $Na_2CO_3$ (99.8% by mass, manufactured by FUJIFILM Wako Pure Chemical Corporation) was dissolved in 1.0 g of ion-exchanged water, and the aqueous solution was dropped little by little at room temperature to 1.0 g of the FeCu carrier. Then the resulting product was dried under vacuum at 60°C overnight. Through the above operations, an Na-FeCu catalyst (Fe : Cu (molar ratio) = 1 : 1, content of Na = 2.0% by mass) was obtained.

[Comparative Example 3: Preparation of Na-Cu/$ZrO_2$ catalyst]

20% Cu/$ZrO_2$ in which CuO is supported on $ZrO_2$ so that the supported amount as CuO is 20% by mass was prepared by polymerized complex method. Specifically, 2.416 g of $Cu(NO_3)_3 \cdot 3H_2O$ (99.0% by mass, manufactured by FUJIFILM Wako Pure Chemical Corporation), 2.30 g of citric acid (99.0% by mass, manufactured by FUJIFILM Wako Pure Chemical Corporation), and 0.148 g of ethylene glycol (99.0% by mass, manufactured by Sigma-Aldrich) were dissolved in 20 mL of ion-exchanged water until uniform. To the resulting aqueous solution, 3.18 g of $ZrO_2$ (98.0% by mass, manufactured by FUJIFILM Wako Pure Chemical Corporation) was added, and the mixture was left to stand overnight with stirring. Then, the resulting product was evaporated to dryness at 120°C with stirring. After drying, the temperature was raised from room temperature (30°C) to 400°C at a raising rate of 3°C/min, and the product was calcined at 400°C for 2 hours to remove the polymer of citric acid and ethylene glycol. Then, the temperature was continuously raised from 400°C to 650°C at a raising rate of 5°C/min, and the product was calcined at 650°C for 5 hours. Thereby, 20% Cu/$ZrO_2$ was obtained.

Then, by IW method, $Na_2CO_3$ was supported on the 20% Cu/$ZrO_2$ so that the content of Na based on the whole of the catalyst would be 2% by mass. Specifically, 0.047 g of $Na_2CO_3$ (99.8% by mass, manufactured by FUJIFILM Wako Pure Chemical Corporation) was dissolved in 1.0 g of ion-exchanged water, and the aqueous solution was dropped little by little at room temperature to 1.0 g of the 20% Cu/$ZrO_2$. Then the resulting product was dried under vacuum at 60°C overnight. Through the above operations, an Na-Cu/$ZrO_2$ catalyst (content of Na = 2.0% by mass) was obtained.

[Comparative Example 4: Preparation of Na-Fe/$ZrO_2$ catalyst]

20% Fe/$ZrO_2$ in which $Fe_2O_3$ is supported on $ZrO_2$ so that the supported amount as $Fe_2O_3$ is 20% by mass was prepared by polymerized complex method. Specifically, 4.02 g of $Fe(NO_3)_3 \cdot 9H_2O$ (99.0% by mass, manufactured by FUJIFILM Wako Pure Chemical Corporation), 1.15 g of citric acid (99.0% by mass, manufactured by FUJIFILM Wako Pure Chemical Corporation), and 0.074 g of ethylene glycol (99.0% by mass, manufactured by Sigma-Aldrich) were dissolved in 20 mL of ion-exchanged water until uniform. To the resulting aqueous solution, 3.194 g of $ZrO_2$ (98.0% by mass, manufactured by FUJIFILM Wako Pure Chemical Corporation) was added, and the mixture was left to stand overnight with stirring. Then, the resulting product was evaporated to dryness at 120°C with stirring. After drying, the temperature was raised from room temperature (30°C) to 400°C at a raising rate of 3°C/min, and the product was calcined at 400°C for 2 hours to remove the polymer of citric acid and ethylene glycol. Then, the temperature was continuously raised from 400°C to 650°C at a raising rate of 5°C/min, and the product was calcined at 650°C for 5 hours. Thereby, 20% FeCu/$ZrO_2$ was obtained.

Then, by IW method, $Na_2CO_3$ was supported on the 20% Fe/$ZrO_2$ so that the content of Na based on the whole of the catalyst would be 2% by mass. Specifically, 0.047 g of $Na_2CO_3$ (99.8% by mass, manufactured by FUJIFILM Wako Pure Chemical Corporation) was dissolved in 1.0 g of ion-exchanged water, and the aqueous solution was dropped little by little at room temperature to 1.0 g of the 20% Fe/$ZrO_2$. Then the resulting product was dried under vacuum at 60°C overnight. Through the above operations, an Na-Fe/$ZrO_2$ catalyst (content of Na = 2.0% by mass) was obtained.

The content of Fe and Cu, the molar ratio $R_{Fe/Cu}$ between Fe and Cu, BET specific surface area $S_{BET}$, pore volume $P_v$, average pore diameter $P_d$, and Cu particle size $d_{Cu}$ for the catalysts of Examples 1 to 4 were measured. The results are shown in Table 1 below.

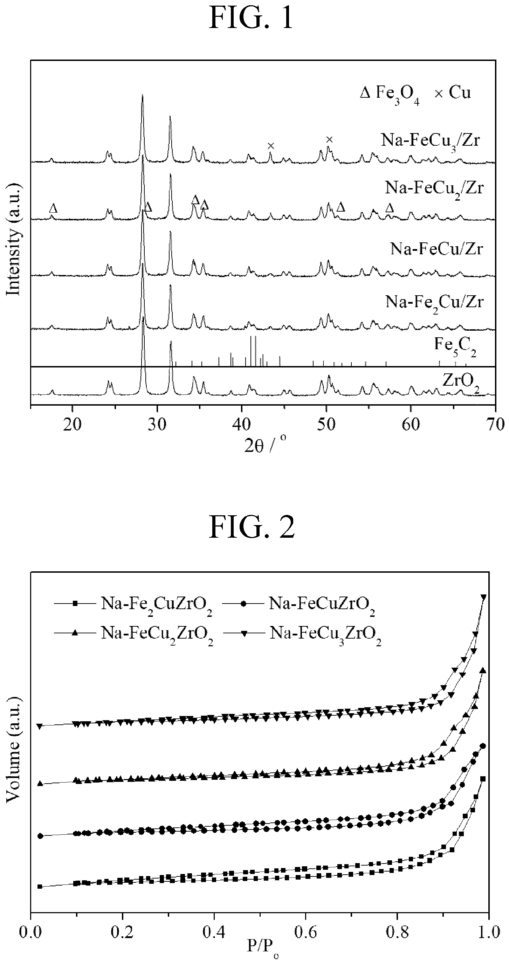

The crystal structures of the catalysts of Examples 1 to 4 were also analyzed using X-ray diffraction (XRD) method (CuK$\alpha$ 40 kV, 20 mA, 10° to 70°). The results are as shown in FIG. 1.

From FIG. 1, the presence of $ZrO_2$ was confirmed for the catalysts of Examples 1 to 4. From the XRF results shown in Table 1, the contents of Fe and Cu were close to the target values, and the Fe/Cu molar ratios were also close to the target values. These results show that Fe and Cu are supported on $ZrO_2$ according to the target value. In view of the above preparation method, it is clear that Na is supported.

The catalyst of Example 1 was subjected to image observation with a scanning electron microscope (SEM), and mapping analysis of Fe, Cu, and Zr was performed by elemental analysis using an EDS (energy dispersive X-ray spectrometer). As a result, it was found that Fe and Cu were highly dispersed on $ZrO_2$.

[Table 1]

| Sample | Content of Fe and Cu (% by mass) | Content as $Fe_2O_3$ and CuO (% by mass) | $R_{Fe/cu}$ | $S_{BET}$ (m²/g) | $P_v$ (cm³/g) | $P_d$ (nm) | $d_{Cu}$ (nm) |
|---|---|---|---|---|---|---|---|
| Ex. 2: Na-$Fe_2$Cu/Zr | 14.5 | 19.8 | 1.9 | 340 | 0.76 | 2.97 | 24.8 |
| Ex. 1: Na-FeCu/Zr | 14.4 | 19.1 | 0.9 | 335 | 1.53 | 3.16 | 28.4 |
| Ex 3: Na-$FeCu_2$/Zr | 16.0 | 20.9 | 0.5 | 334 | 0.87 | 3.13 | 39.8 |
| Ex 4: Na-$FeCu_3$/Zr | 16.8 | 21.7 | 0.3 | 307 | 0.63 | 3.12 | 49.6 |

As shown in Table 1, when Fe/Cu = 2/1 (Example 2), the BET specific surface area was the largest, $d_{Cu}$ was the smallest, and Cu was most highly dispersed. Meanwhile, the average pore diameter $P_d$ was the largest when Fe/Cu = 1/1 (Example 1). Presumably, the larger the pore diameter, the easier it is for gas to pass through the pores, and the $CO_2$ conversion is likely to improve.

Each adsorption isotherm of the catalysts of Examples 1 to 4 was determined. The adsorption isotherm was measured using a BET specific surface area measuring apparatus (NOVA2200e, manufactured by Quantachrome). Into a 9 mL cell, 0.02 to 0.05 g of the powdered catalyst was placed and pretreated at 200°C for 2 hours under vacuum. Then, measurement was performed by 79-point method using nitrogen as an adsorption gas. The results are as shown in FIG. 2, and no abnormality was observed in the adsorption isotherms of Examples 1 to 4. Thus, the values of BET specific surface area, pore volume, and average pore diameter in Table 1 were highly reliable.

The catalysts of Examples 1 to 4 and Comparative Examples 3 and 4 were subjected to the measurement by temperature programmed reduction ($H_2$-TPReduction measurement) using hydrogen as a reducing gas. BELCAT II-T-SP (manufactured by MicrotracBEL Corp.) was used for the measurement by temperature programmed reduction. A TCD was used as a detector, and the measurement was performed in the range from room temperature to 600°C

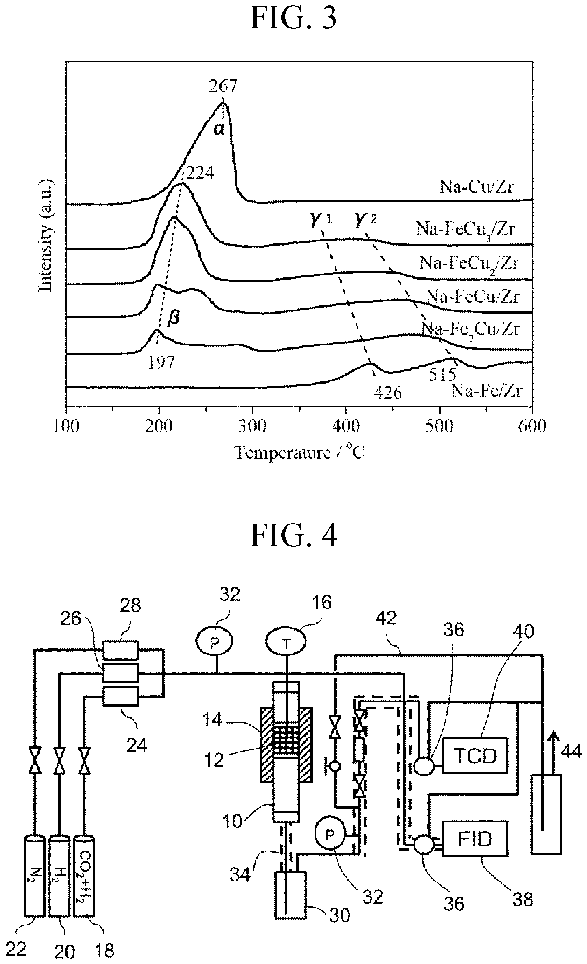

The results are shown in FIG. 3. In FIG. 3, $\alpha$, $\beta$, $\gamma_1$, and $\gamma_2$ represent the following reactions, respectively.

$\alpha$:

$$CuO \rightarrow Cu$$

$\beta$:

$$CuFe_2O_4 \rightarrow Cu + Fe_3O_4$$

$\gamma_1$:

$$Fe_2O_3 \rightarrow Fe_3O_4$$

$\gamma_2$:

$$Fe_3O_4 \rightarrow FeO + Fe$$

As shown in FIG. 3, when compared among Examples 1 to 4, the higher the molar ratio of Fe, the lower the reduction temperature of $\beta$. The higher the molar ratio of Cu, the lower the reduction temperature of $\gamma_1$ and $\gamma_2$. It means that the lower the reduction temperature, the higher the dispersion of the oxide before reduction. Thus, presumably, Example 1 (Na-FeCu/$ZrO_2$) and Example 3 (Na-$FeCu_2$/$ZrO_2$), which are located in the middle among Examples 1 to 4, are more preferable for the molar ratio of Fe and Cu.

[Examples 5 to 7: Preparation of Catalysts with Different Supported Amounts of Fe and Cu]

Catalysts of Examples 5 to 7 were prepared in the same manner as in Example 1 except that the amount of $ZrO_2$ added in polymerized complex method was changed so that the supported amount of $Fe_2O_3$ and CuO would be 40% by mass, 30% by mass, or 10% by mass while maintaining the Fe/Cu molar ratio at 1/1. Specifically, in Example 5, the addition amount of $ZrO_2$ was 1.196 g, and an Na-40% FeCu/$ZrO_2$ catalyst (content of Na = 2.0% by mass) having a supported amount as $Fe_2O_3$ and CuO of 40% by mass was obtained. In Example 6, the addition amount of $ZrO_2$ was 1.86 g, and an Na-30% FeCu/$ZrO_2$ catalyst (content of Na = 2.0% by mass) having a supported amount as $Fe_2O_3$ and CuO of 30% by mass was obtained. In Example 7, the addition amount of $ZrO_2$ was 7.17 g, and an Na-10% FeCu/$ZrO_2$ catalyst (content of Na = 2.0% by mass) having a supported amount as $Fe_2O_3$ and CuO of 10% by mass was obtained.

[Examples 8 and 9 and Comparative Example 5: Preparation of Na-FeCu/$TiO_2$, $Al_2O_3$, and $SiO_2$ catalysts]

Catalysts of Examples 8 and 9 and Comparative Example 5 were prepared in the same manner as in Example 6 except that 1.86 g of $TiO_2$ (99.0% by mass, manufactured by FUJIFILM Wako Pure Chemical Corporation) was used in Example 8, 1.86 g of $Al_2O_3$ (99.0% by mass, manufactured by Sigma-Aldrich) was used in Example 9, and 1.86 g of $SiO_2$ (99.0% by mass, manufactured by Sigma-Aldrich) was used in Comparative Example 5 instead of $ZrO_2$ as the carrier powder. The catalyst of Example 8 is an Na-FeCu/$TiO_2$ catalyst. The catalyst of Example 9 is an Na-FeCu/$Al_2O_3$ catalyst. The catalyst of Comparative Example 5 is an Na-FeCu/$SiO_2$ catalyst.

[Example 10 and Comparative Example 6: Preparation of K- and Cs-FeCu/$ZrO_2$ catalysts]

Each catalyst of Example 10 and Comparative Example 6 was prepared in the same manner as in Example 1 except that in the supporting step by IW method, 0.036 g of $K_2CO_3$ (99.5% by mass, manufactured by FUJIFILM Wako Pure Chemical Corporation) was used in Example 10, and 0.025 g of $Cs_2CO_3$ (98.0% by mass, manufactured by KANTO KAGAKU) was used in Comparative Example 6 instead of 0.047 g of $Na_2CO_3$. The catalyst of Example 10 is a K-FeCu/$ZrO_2$ catalyst. The catalyst of Comparative Example 6 is a Cs-FeCu/$ZrO_2$ catalyst.

[Catalyst Performance Evaluation Test Method]

The catalysts prepared above were subjected to a catalyst performance evaluation test by a synthesis reaction of ethanol from carbon dioxide. The evaluation test was performed using the reactor shown in FIG. 4.
In FIG. 4, a reference sign 10 indicates a fixed bed flow reactor, a reference sign 12 indicates a catalyst bed in the reactor 10, a reference sign 14 indicates a heater for heating the reactor 10, and a reference sign 16 indicates a thermometer for sensing the temperature of the catalyst bed 12. A reference sign 18 indicates a cylinder that supplies $CO_2$ and $H_2$ as a reaction gas, a reference sign 20 indicates a cylinder that supplies $H_2$ as a reducing gas, a reference sign 22 indicates a cylinder that supplies a nitrogen gas, and mass flow controllers (MFCs) 24, 26, and 28 are connected to cylinders 18, 20, and 22, respectively. A reference sign 30 is an ice trap provided downstream of the reactor 10, and serves to cool long-chain hydrocarbons and alcohol products that can clog piping and recover them as liquids. Reference signs 32 indicate pressure gauges that detect the pressure of the reaction system, and are provided upstream and downstream of the reactor 10. A reference sign 34 indicates a ribbon heater as a line heating that heats the piping downstream of the reactor 10, and a reference sign 36 indicates a hexagonal valve. A reference sign 38 indicates a GC-FID (flame ionization detector) for detecting organic compounds, and a reference sign 40 indicates a GC-TCD (thermal conductivity detector) for detecting other compounds. A reference sign 42 indicates a bypass pathway, and a reference sign 44 indicates an exhaust gas outlet.
The catalyst (0.5 g) and quartz sand (1.0 g) were mixed and packed in the reactor 10 to form the catalyst bed 12, which was fixed by placing quartz wool on both sides. The catalyst was reduced. Specifically, $H_2$ gas was introduced into the reaction system from the cylinder 20 and flowed into the reactor 10 at 60 mL/min, and reduction was performed at 400°C and 0.1 MPa for 8 hours.
Then, the supply of $H_2$ gas was stopped, and $H_2$ gas and $CO_2$ gas were supplied as a reaction gas from the cylinder 18 at a $H_2/CO_2$ volume ratio of 3/1. The reaction gas was flowed into the reactor 10 at 17 mL/min (W/F = 35 g·h/mol), and the reaction was performed at 320°C and 5.0 MPa for 8 hours (Time on Stream (TOS) = 8 h).
The flow pass was switched by the hexagonal valve 36 every 2 hours from the start of the reaction, and the gas components produced during the reaction were analyzed by the online GC-FID 38 and the online GC-TCD 40. The liquid components in the ice trap 30 collected after the reaction was completed were analyzed by an offline GC-FID (not shown). As the online GC-FID 38, GC-2014 (manufactured by Shimadzu Corporation) was used, and as the column, active carbon (carrier: nitrogen, manufactured by GL Sciences Inc.) was used. As the online GC-TCD 40, GC-2014 (manufactured by Shimadzu Corporation) was used, and as the column, PorapakQ (carrier: helium, manufactured by GL Sciences Inc.) was used. As the offline GC-FID, GC-2014 (manufactured by Shimadzu Corporation) was used, and as the column, InertCap5 (carrier: nitrogen, manufactured by GL Sciences Inc.) was used.

From the results of analysis above, the $CO_2$ conversion, CO selectivity, and percentage of each product were determined. The $CO_2$ conversion is the percentage of the $CO_2$ converted to CO, hydrocarbons, and alcohols (i.e. all the products) based on the $CO_2$ supplied to the reaction system. The CO selectivity is the percentage of CO remaining without being converted to hydrocarbons or alcohols based on CO produced by conversion. The percentage of each product is the mass percentage of each product in all the products (hydrocarbons and alcohols) other than CO.

Calculating formulas for these are shown below. As for the percentage of each product, formulas for calculating the percentage of the target hydrocarbon and the percentage of the target alcohol are shown. The peak areas of each product and an internal standard detected by the online GC-TCD, GC-FID and offline GC-FID were applied to the following formulas, and each calculation was performed. To the reaction gas, 3.04% by volume of argon gas (internal standard) was added and was used as the internal standard for TCD. A few grams of decane was added to analyze the organic phase (hydrocarbon) of FID, a few grams of 2-butanol was added to analyze the aqueous phase (alcohol), and these were used as internal standards.

[Mathematical Expression 1]

$$CO_2 \text{ conversion } \% = \frac{\left(\dfrac{CO_2 \text{ area before reaction}}{Ar \text{ area before reaction}}\right) - \left(\dfrac{CO_2 \text{ area after reaction}}{Ar \text{ area after reaction}}\right)}{\left(\dfrac{CO_2 \text{ area before reaction}}{Ar \text{ area before reaction}}\right)} \times 100$$

The $CO_2$ area before reaction is the value of the peak area of $CO_2$ detected when the unreacted gas is analyzed by online GC-TCD. The $CO_2$ area after reaction is the value of the peak area of $CO_2$ detected when the gas product after reaction is analyzed by the online GC-TCD. The Ar area before reaction is the value of the peak area of Ar (internal standard) detected when the unreacted gas is analyzed by the online GC-TCD. The Ar area after reaction is the value of the peak area of Ar (internal standard) detected when the gas product after the reaction is analyzed by the online GC-TCD.

[Mathematical Expression 2]

$$CO \text{ selectivity } \% = \frac{\text{Produced CO } [mol/g \cdot h]}{\text{Amount of converted } CO_2 \ [mol/g \cdot h]} \times 100$$

The amount of produced CO and the amount of converted $CO_2$ are as follows, and the amount of produced gas in the produced CO and the produced CO ratio are as follows.

[Mathematical Expression 3]

$$\text{Produced CO [mol/g} \cdot \text{h]} = \frac{\text{Amount of produced gas [mol/h]} \times \text{Produced CO ratio}}{\text{Amount of catalyst } w\text{[g]}}$$

Amount of converted $CO_2$ [mol/g $\cdot$ h]

$$= \left( \frac{\text{Atmospheric pressure } P[\text{Pa}] \times \text{inlet velocity } F_0[\text{mL/min}] \times \frac{60}{1000}}{8.315[\text{Pa} \cdot \text{m}^3/\text{K} \cdot \text{mol}] \times \text{Temperature of room } T[\text{K}] \times 1000} \right) [\text{mol/h}]$$

$$\times \frac{\text{CO}_2 \text{ composition (of reaction gas) before reaction}}{100} \times \frac{\left(\frac{\text{CO}_2 \text{ conversion \%}}{100}\right)}{\text{Amount of catalyst } w \text{ [g]}}$$

Amount of produced gas [mol/h]

$$= \left( \frac{10}{\text{Outlet velocity } F[\text{s/10mL}]} \times \frac{3600}{1000} \right) [\text{L/h}]$$

$$\times \left( \frac{\text{Atmospheric pressure } P[\text{Pa}]}{8.315[\text{Pa} \cdot \text{m}^3/\text{K} \cdot \text{mol}] \times \text{Temperature of room } T[\text{K}]} \times \frac{1}{1000} \right) [\text{mol/L}]$$

$$\text{Produced CO ratio} = \frac{\text{CO composition ratio of standard gas}}{100} \times \frac{\text{CO area after reaction}}{\text{CO area of standard gas}}$$

The CO area of the standard gas was determined by analyzing the gas in the standard gas cylinder before reaction by the online GC-TCD through a bypass without passage through the reactor, and calculating the peak area. The volume ratio of standard gas was $H_2 : CO : CH_4 : CO_2 = 85.55 : 4.8 : 4.95 : 4.7$.

[Mathematical Expression 4]

$$\text{Percentage \% of target hydrocarbon} = \left( \frac{\text{D} + \text{E}}{\text{A} + \text{B} + \text{C}} \right) \times 100$$

$$\text{Percentage \% of target alcohol} = \left( \frac{\text{F}}{\text{A} + \text{B} + \text{C}} \right) \times 100$$

A et F in the formulae are as follows.

A: Total hydrocarbon detected by the online FID [mol/g h]
B: Total hydrocarbon detected by the offline FID [mol/g·h]
C: Total alcohol detected by the offline FID [mol/g·h]
D: Target hydrocarbon detected by the online FID [mol/g·h]
E: Target hydrocarbon detected by the offline FID [mol/g·h]
F: Target alcohol detected by the offline FID [mol/g·h]

The number of moles (mol/g·h) of hydrocarbons (having 1 to 6 carbon atoms) detected by the online FID per 1 hour of reaction per 1 g of the catalyst is determined by the following formula (produced $CH_4$ ratio in the formula is as follows).

[Mathematical Expression 5]

Hydrocarbon $[mol/g \cdot h]$

$$= \frac{\text{Peak area of target hydrocarbon}}{\text{Peak area of } CH_4} \times \text{Amount of produced gas } [mol/h]$$

$$\times \text{Produced } CH_4 \text{ ratio} \times \frac{1}{\text{Amount of catalyst } w[g]}$$

Produced $CH_4$ ratio

$$= \frac{CH_4 \text{ composition ratio of standard gas}}{100} \times \frac{CH_4 \text{ area after reaction}}{CH_4 \text{ area of standard gas}}$$

The number of moles (mol/g h) of hydrocarbons (having 4 or more carbon atoms) and alcohols detected by the offline FID per 1 hour of reaction per 1 g of the catalyst is determined by the following formulae.

[Mathematical Expression 6]

Hydrocarbon $[mol/g \cdot h]$

$$= \frac{\text{Peak area of target hydrocarbon}}{\text{Peak area of internal standard (decane)}}$$

$$\times \frac{\text{Carbon number of internal standard (decane)} \times \text{Addition amount of internal standard (decane) } [mol]}{\text{Amount of catalyst } w[g] \times \text{Reaction time } t[h]}$$

Alcohol $[mol/g \cdot h]$

$$= \frac{\text{Peak area of target alcohol}}{\text{Peak area of internal standard (butanol)}}$$

$$\times \frac{\text{Carbon number of internal standard (butanol)} \times \text{Addition amount of internal standard (butanol) } [mol]}{\text{Amount of catalyst } w[g] \times \text{Reaction time } t[h]}$$

The yield of ethanol (% by mass) relative to the $CO_2$ charged and the ethanol selectivity (% by mass) which is the percentage of ethanol in all the products including CO were calculated by the following formulae.

[Mathematical Expression 7]

$$\text{Ethanol yield \%} = \frac{\text{CO}_2 \text{ conversion \%}}{100} \times \frac{100 - \text{CO selectivity \%}}{100} \times \text{Ethanol percentage \%}$$

$$\text{Ethanol selectivity \%} = \text{Ethanol percentage \%} \times \frac{100 - \text{CO selectivity \%}}{100}$$

The STY of ethanol was calculated by the following formula. The STY is called Space Time Yield, and represents the amount (mg) of the target product (ethanol) produced per 1 hour of reaction per 1 g of catalyst.

[Mathematical Expression 8]

$$\text{STY } [\text{mg/g}_{\text{cat}} \cdot \text{h}]$$

$$= \left\{ \frac{\text{Outlet velocity } F \text{ [L/h]}}{22.4 \text{ L/mol}} \times \text{CO}_2 \text{ composition ratio of reaction gas} \right.$$

$$\times \left(1 - \frac{\text{CO selectivity}}{100}\right) \times \frac{\text{CO}_2 \text{ conversion}}{100} \times \left. \frac{\text{Ethanol percentage \%}}{100} \right\}$$

$$\times \text{Molecular weight of ethanol } [\text{m g/mol}] \times \frac{1}{2} \times \frac{1}{\text{Amount of catalyst } w [\text{g}]}$$

[Catalyst Performance Evaluation of Examples 1 to 4]

A catalyst performance evaluation test was performed on each catalyst of Examples 1 to 4 and Comparative Examples 1 to 4. The results are shown in Table 2 below. In Table 2, in "Hydrocarbon distribution", "$CH_4$" represents the percentage % of methane, "$C_{2\ to\ 4}$" represents the percentage % of hydrocarbons having 2 to 4 carbon atoms, and "$C_{5\ to\ 11}$" represents the percentage % of hydrocarbons having 5 to 11 carbon atoms, and "$C_{12+}$" represents the percentage % of hydrocarbons having 12 or more carbon atoms. In the "Alcohol distribution", "$C_1$" represents the percentage % of methanol, "$C_2$" represents the percentage % of methanol, "$C_3$" represents the percentage % of propyl alcohol, and "$C_{4+}$" represents the percentage % of alcohols having 4 or more carbon atoms. The same applies to Tables 3 to 5.

[Table 2]

| | CO$_2$ Conversion [%] | CO Selectivity [%] | Hydrocarbon distribution [%] | | | |
| --- | --- | --- | --- | --- | --- | --- |
| | | | CH$_4$ | C$_{2\ to\ 4}$ | C$_{5\ to\ 11}$ | C$_{12+}$ |
| Comp. Ex. 1: Na-Fe | 32.4 | 11.9 | 11.6 | 33.0 | 39.9 | 1.6 |
| Comp. Ex. 2: Na-FeCu | 30.4 | 15.4 | 7.9 | 22.6 | 38.8 | 4.7 |
| Comp. Ex. 3: Na-Cu/ZrO$_2$ | 24.1 | 81.2 | 4.6 | 4.6 | 4.5 | 0.5 |
| Comp. Ex. 4: Na-Fe/ZrO$_2$ | 33.6 | 17.8 | 19.4 | 38.2 | 15.9 | 1.1 |
| Ex. 2: Na-Fe$_2$Cu/ZrO$_2$ | 38.6 | 10.9 | 9.2 | 22.2 | 29.1 | 2.2 |
| Ex. 1: Na-FeCu/ZrO$_2$ | 38.7 | 7.1 | 7.6 | 19.7 | 21.1 | 2.2 |
| Ex. 3: Na-FeCu$_2$/ZrO$_2$ | 40.0 | 9.2 | 8.8 | 20.8 | 24.9 | 0.7 |

(continued)

| | CO$_2$ Conversion [%] | CO Selectivity [%] | Hydrocarbon distribution [%] | | | |
|---|---|---|---|---|---|---|
| | | | CH$_4$ | C$_{2 \text{ to } 4}$ | C$_{5 \text{ to } 11}$ | C$_{12+}$ |
| Ex. 4: Na-FeCu$_3$/ZrO$_2$ | 38.0 | 10.1 | 7.9 | 19.4 | 26.7 | 1.7 |

| | Alcohol distribution [%] | | | | Ethanol | |
|---|---|---|---|---|---|---|
| | C1 | C$_2$ | C$_3$ | C$_{4+}$ | Yield [%] | Selectivity [%] |
| Comp. Ex. 1: Na-Fe | 2.0 | 7.7 | 1.4 | 2.6 | 2.20 | 6.8 |
| Comp. Ex. 2: Na-FeCu | 1.7 | 15.8 | 3.6 | 5.0 | 4.06 | 13.4 |
| Comp. Ex. 3: Na-Cu/ZrO$_2$ | 82.7 | 2.0 | 0.7 | 0.4 | 0.09 | 0.4 |
| Comp. Ex. 4: Na-Fe/ZrO$_2$ | 1.5 | 17.7 | 2.9 | 3.3 | 4.89 | 14.5 |
| Ex. 2: Na-Fe$_2$Cu/ZrO$_2$ | 2.8 | 27.2 | 4.2 | 3.1 | 9.35 | 24.2 |
| Ex. 1: Na-FeCu/ZrO$_2$ | 3.9 | 35.6 | 5.6 | 4.2 | 12.80 | 33.1 |
| Ex. 3: Na-FeCu$_2$/ZrO$_2$ | 3.5 | 33.2 | 4.6 | 3.5 | 12.06 | 30.1 |
| Ex. 4: Na-FeCu$_3$/ZrO$_2$ | 3.9 | 32.6 | 4.4 | 3.4 | 11.14 | 29.3 |

As shown in Table 2, in Examples 1 to 4 in which Fe and Cu were supported on ZrO$_2$, the CO$_2$ conversion and ethanol selectivity were improved, and the yield of ethanol was high compared to Comparative Examples 1 to 4. In particular, Example 1 achieved a CO$_2$ conversion of 38.7% and an ethanol selectivity of 33.1% (percentage of ethanol of 35.6% in the total hydrocarbon and alcohol product excluding CO).

In the catalysts of Examples 1 to 4, Fe and Cu are supported as Fe$_2$O$_3$ and CuO, and Na is supported as Na$_2$CO$_3$. Presumably, Fe$_2$O$_3$ was reduced to Fe$^0$ and Fe$_3$O$_4$, and CuO was reduced to Cu$^0$ by subjecting the catalysts to the reduction treatment. Presumably, Fe$^0$, Fe$_3$O$_4$, and Cu$^0$ supported on ZrO$_2$ promote the conversion of CO$_2$ to ethanol by supply of the reaction gas in this state to cause reaction. Specifically, Fe$_3$O$_4$ and Cu$^0$ on ZrO$_2$ promoted the conversion of CO$_2$ to CO (RWGS), and the CO$_2$ conversion was improved. Fe$_3$O$_4$ produced Fe$_5$C$_2$ through the reduction reaction with CO produced by RWGS, Fe$^0$, Fe$_5$C$_2$, and Cu$^0$ on ZrO$_2$ promoted ethanol synthesis from CO, and the ethanol selectivity was improved.

[Catalyst Performance Evaluation of Examples 5 to 7]

The results of a catalyst performance evaluation test performed on the catalysts of Examples 1 and 5 to 7 to compare the catalyst performances for different supported amounts of Fe and Cu are shown in Table 3 below.

[Table 3]

| | CO$_2$ Conversion [%] | CO Selectivity [%] | Hydrocarbon distribution [%] | | | |
|---|---|---|---|---|---|---|
| | | | CH$_4$ | C$_{2 \text{ to } 4}$ | C$_{5 \text{ to } 11}$ | C$_{12+}$ |
| Ex. 5: Na-40%FeCu/ZrO$_2$ | 37.0 | 12.6 | 8.1 | 22.6 | 28.9 | 1.1 |
| Ex. 6: Na-30%FeCu/ZrO$_2$ | 38.2 | 9.9 | 9.7 | 21.6 | 21.9 | 0.9 |
| Ex. 1: Na-20%FeCu/ZrO$_2$ | 38.7 | 7.1 | 7.6 | 19.7 | 21.1 | 2.2 |
| Ex. 7: Na-10%FeCu/ZrO$_2$ | 36.6 | 13.2 | 12.5 | 27.1 | 19.2 | 0.4 |

(continued)

| | Alcohol distribution [%] | | | | Ethanol | |
|---|---|---|---|---|---|---|
| | $C_1$ | $C_2$ | $C_3$ | $C_{4+}$ | Yield [%] | Selectivity [%] |
| Ex. 5: Na-40%FeCu/ZrO$_2$ | 3.7 | 29.2 | 4.1 | 2.3 | 9.44 | 25.5 |
| Ex. 6: Na-30%FeCu/ZrO$_2$ | 4.2 | 34.2 | 4.9 | 2.6 | 11.77 | 30.8 |
| Ex. 1: Na-20%FeCu/ZrO$_2$ | 3.9 | 35.6 | 5.6 | 4.2 | 12.80 | 33.1 |
| Ex. 7: Na-10%FeCu/ZrO$_2$ | 3.5 | 31.0 | 4.2 | 2.1 | 9.85 | 26.9 |

As shown in Table 3, even when the supported amounts of Fe and Cu on $ZrO_2$ were changed, the $CO_2$ conversions and ethanol selectivities were high, and the yields of ethanol were high. In particular, Examples 1 and 6, in which the supported amounts as $Fe_2O_3$ and CuO were 20 to 30% by mass, were excellent in selectivity and yield of ethanol.

[Catalyst Performance Evaluation of Examples 8 and 9]

The results of a catalyst performance evaluation test performed on the catalysts of Examples 6, 8, 9 and Comparative Example 5 to compare the catalyst performances for different carriers are shown in Table 4 below.

[Table 4]

| | CO$_2$ Conversion [%] | CO Selectivity [%] | Hydrocarbon distribution [%] | | | |
|---|---|---|---|---|---|---|
| | | | CH$_4$ | C$_{2 \text{ to } 4}$ | C$_{5 \text{ to } 11}$ | C$_{12+}$ |
| Comp. Ex. 5: Na-FeCu/SiO$_2$ | 22.8 | 81.5 | 12.5 | 3.7 | 6.2 | 0.1 |
| Ex. 8: Na-FeCu/TiO$_2$ | 35.4 | 12.5 | 13.7 | 31.6 | 24.9 | 0.4 |
| Ex. 9: Na-FeCu/Al$_2$O$_3$ | 38.6 | 4.5 | 5.5 | 13.3 | 41.9 | 4.7 |
| Ex. 6: Na-FeCu/ZrO$_2$ | 38.2 | 9.9 | 9.7 | 21.6 | 21.9 | 0.9 |
| | Alcohol distribution [%] | | | | Ethanol | |
| | C1 | C$_2$ | C$_3$ | C4, | Yield [%] | Selectivity [%] |
| Comp. Ex. 5: Na-FeCu/SiO$_2$ | 75.6 | 1.4 | 0.3 | 0.2 | 0.06 | 0.03 |
| Ex. 8: Na-FeCu/TiO$_2$ | 2.7 | 21.9 | 3.4 | 1.4 | 6.78 | 19.2 |
| Ex. 9: Na-FeCu/Al$_2$O$_3$ | 2.2 | 25.1 | 3.9 | 3.4 | 9.25 | 24.0 |
| Ex. 6: Na-FeCu/ZrO$_2$ | 4.2 | 34.2 | 4.9 | 2.6 | 11.77 | 30.8 |

As shown in Table 4, in Comparative Example 5, in which $SiO_2$ was used as a carrier, not only was the $CO_2$ conversion low, but also the yield of ethanol was low due to the low conversion of CO to alcohols and hydrocarbons. Meanwhile, in Example 8, in which $TiO_2$ was used as a carrier, and in Example 9, in which $Al_2O_3$ was used as a carrier, the $CO_2$ conversion and ethanol selectivity were high, and the yield of ethanol was high same as in Example 6, in which $ZrO_2$ was used. Example 6, in which $ZrO_2$ was used as a carrier, showed the best results.

[Catalyst Performance Evaluation of Example 10]

The results of a catalyst performance evaluation test performed on the catalysts of Examples 1 and 10, and Comparative Example 6 to compare the catalyst performances for different alkali metals are shown in Table 5 below.

[Table 5]

| | $CO_2$ Conversion [%] | CO Selectivity [%] | Hydrocarbon distribution [%] | | | |
| --- | --- | --- | --- | --- | --- | --- |
| | | | $CH_4$ | $C_{2\ to\ 4}$ | $C_{5\ to\ 11}$ | $C_{12+}$ |
| Comp. Ex. 6: Cs-FeCu/$ZrO_2$ | 35.8 | 13.6 | 21.9 | 33.5 | 16.9 | 0.6 |
| Ex. 10: K-FeCu/$ZrO_2$ | 37.9 | 12.2 | 9.7 | 19.1 | 30.0 | 2.5 |
| Ex. 1: Na-FeCu/$ZrO_2$ | 38.7 | 7.1 | 7.6 | 19.7 | 21.1 | 2.2 |
| | Alcohol distribution [%] | | | | Ethanol | | STY [mg/ ($g_{cat}$·h)] |
| | $C_1$ | $C_2$ | $C_3$ | $C_{4+}$ | Yield [%] | Selectivity [%] | |
| Comp. Ex. 6: Cs-FeCu/$ZrO_2$ | 5.0 | 15.5 | 4.5 | 2.1 | 4.79 | 13.4 | 9.0 |
| Ex. 10: K-FeCu/$ZrO_2$ | 3.2 | 28.1 | 4.1 | 3.3 | 9.35 | 27.4 | 17.6 |
| Ex. 1: Na-FeCu/$ZrO_2$ | 3.9 | 35.6 | 5.6 | 4.2 | 12.80 | 33.1 | 24.6 |

As shown in Table 5, Na was the best as the alkali metal to be supported on FeCu/$ZrO_2$, and even in Example 10, in which K was supported, the $CO_2$ conversion and ethanol selectivity were high, and the yield of ethanol was high.

[Investigation of reaction conditions]

The catalyst performance was evaluated in the same manner as described above except that the temperature and W/F as reaction conditions were changed as shown in Table 6 below in the implementation of the catalyst performance evaluation test using the catalyst of Example 1.

The W/F is the contact time that represents the degree of load on the catalyst, W is the mass of the catalyst [g], F is the molar flow rate [mol/h] of the reaction gas, and the unit is g·h/mol. A smaller W/F means a higher flow velocity.

[Table 6]

| Reaction temperature [°C] | W/F [g·h/mol] | $CO_2$ Conversion [%] | CO Selectivity [%] | Hydrocarbon distribution [%] | | | |
| --- | --- | --- | --- | --- | --- | --- | --- |
| | | | | $CH_4$ | $C_{2\ to\ 4}$ | $C_{5\ to\ 11}$ | $C_{12+}$ |
| 300 | 35 | 35.8 | 9.5 | 9.1 | 21.5 | 21.2 | 1.3 |
| 340 | 35 | 42.3 | 9.4 | 6.6 | 20.7 | 22.1 | 0.8 |
| 320 | 5 | 30.0 | 21.4 | 10.2 | 27.4 | 18.2 | 1.3 |
| 320 | 15 | 35.6 | 11.2 | 7.4 | 19.1 | 20.5 | 1.6 |
| 320 | 35 | 38.7 | 8.8 | 8.6 | 19.2 | 21.5 | 0.7 |
| Reaction temperature [°C] | W/F [g·h/mol] | Alcohol distribution [%] | | | | Ethanol selectivity [%] | STY [mg/ ($g_{cat}$·h)] |
| | | $C_1$ | $C_2$ | $C_3$ | $C_{4+}$ | | |
| 300 | 35 | 3.0 | 33.0 | 5.6 | 5.4 | 29.9 | 18.7 |
| 340 | 35 | 2.7 | 32.9 | 8.0 | 6.1 | 29.8 | 25.4 |
| 320 | 5 | 2.3 | 26.8 | 5.3 | 8.5 | 21.1 | 79.6 |
| 320 | 15 | 2.8 | 35.8 | 6.4 | 6.4 | 31.8 | 46.8 |
| 320 | 35 | 3.1 | 35.1 | 6.6 | 5.1 | 32.0 | 24.1 |

As shown in Table 6, 320°C was the best as the reaction temperature because the $CO_2$ conversion was improved by raising the reaction temperature, but the ethanol selectivity was lowered. As the flow velocity increased (W/F decreased), the amount of ethanol produced (STY) per 1 hour of reaction per 1 g of catalyst increased, but the $CO_2$ conversion and ethanol selectivity decreased. The W/F is preferably 3 to 50 g·h/mol, more preferably 5 to 45 g·h/mol, and further preferably 10 to 40 g - h/mol.

As described above, according to the embodiment, the improvement of the $CO_2$ conversion and the highly selective synthesis of ethanol can be achieved in the direct synthesis reaction of ethanol from $CO_2$. Thus, the embodiment is useful as a means for synthesizing ethanol from $CO_2$.

Although some embodiments of the invention have been described above, these embodiments are presented by way of example and are not intended to limit the scope of the invention. These embodiments can be implemented in various other forms, and various omissions, replacements, and modifications can be made without departing from the gist of the invention. These embodiments, omissions, replacements, modifications and the like thereof are included in the invention described in claims and equivalents thereof, as well as being included in the scope and gist of the invention.

## Claims

1. An ethanol synthesis catalyst for synthesizing ethanol from carbon dioxide, comprising:

   at least one carrier selected from the group consisting of $ZrO_2$, $Al_2O_3$, and $TiO_2$;
   Fe and Cu supported on the carrier; and
   Na and/or K supported on the carrier.

2. The ethanol synthesis catalyst according to claim 1, comprising iron oxide and copper oxide as the Fe and Cu supported on the carrier.

3. The ethanol synthesis catalyst according to claim 1, comprising at least one selected from the group consisting of $Fe^0$ and $Fe_3O_4$ as the Fe supported on the carrier, and $Cu^0$ as the Cu supported on the carrier.

4. The ethanol synthesis catalyst according to any one of claims 1 to 3, comprising at least one selected from the group consisting of $Na_2CO_3$, $K_2CO_3$, NaCl, KCl, NaOH, KOH, $NaNO_3$, $KNO_3$, $Na_2SO_4$, and $K_2SO_4$ as the Na and/or K supported on the carrier.

5. The ethanol synthesis catalyst according to any one of claims 1 to 4, wherein a content of Fe and Cu is 5 to 35% by mass.

6. The ethanol synthesis catalyst according to any one of claims 1 to 5, wherein a molar ratio Fe/Cu between Fe and Cu is 0.1 to 2.5.

7. The ethanol synthesis catalyst according to any one of claims 1 to 6, wherein a content of Na and/or K is 0.1 to 10% by mass.

8. A production method of ethanol, comprising:
   obtaining ethanol from carbon dioxide in the presence of a catalyst including at least one carrier selected from the group consisting of $ZrO_2$, $Al_2O_3$, and $TiO_2$; Fe and Cu supported on the carrier; and Na and/or K supported on the carrier.

9. The production method of ethanol according to claim 8, wherein the catalyst including iron oxide and copper oxide as the Fe and Cu supported on the carrier is used, hydrogen is supplied to the catalyst to reduce iron oxide and copper oxide, and a reaction gas containing carbon dioxide is supplied to the catalyst after reduction to obtain ethanol from carbon dioxide.

10. The production method of ethanol according to claim 9, wherein the catalyst includes $Fe_2O_3$ in a state before reduction, includes at least one selected from the group consisting of $Fe^0$ and $Fe_3O_4$ in a reduced state as the Fe supported on the carrier, and includes CuO in a state before reduction, and includes $Cu^0$ in a reduced state as the Cu supported on the carrier.

11. The production method of ethanol according to any one of claims 8 to 10, wherein the catalyst includes at least one

EP 4 331 722 A1

selected from the group consisting of $Na_2CO_3$, $K_2CO_3$, NaCl, KCl, NaOH, KOH, $NaNO_3$, $KNO_3$, $Na_2SO_4$, and $K_2SO_4$ as the Na and/or K supported on the carrier.

# FIG. 1

# FIG. 2

# FIG. 3

# FIG. 4

**INTERNATIONAL SEARCH REPORT**

| International application No. |
| --- |
| PCT/JP2021/016831 |

A.  CLASSIFICATION OF SUBJECT MATTER
B01J 23/745(2006.01)i; C07C 29/156(2006.01)i; C07C 31/08(2006.01)i
FI: B01J23/745 M; C07C29/156; C07C31/08

According to International Patent Classification (IPC) or to both national classification and IPC

B.  FIELDS SEARCHED

Minimum documentation searched (classification system followed by classification symbols)
B01J23/745; C07C29/156; C07C31/08

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY (STN)

C.  DOCUMENTS CONSIDERED TO BE RELEVANT

| Category* | Citation of document, with indication, where appropriate, of the relevant passages | Relevant to claim No. |
| --- | --- | --- |
| X | JP 2008-247778 A (TOKYO ELECTRIC POWER CO INC) 16 October 2008 (2008-10-16) claims 1-12, paragraphs [0018], [0032] | 1-3, 8-10 |
| A | JP 2020-011228 A (TOYOTA CENTRAL RES & DEV) 23 January 2020 (2020-01-23) claims 1-7 | 1-11 |
| A | CN 102145287 A (HARBIN INSTITUTE OF TECHNOLOGY) 10 August 2011 (2011-08-10) entire text | 1-11 |

☐ Further documents are listed in the continuation of Box C.   ☒ See patent family annex.

| | |
| --- | --- |
| * Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| 16 June 2021 (16.06.2021) | 29 June 2021 (29.06.2021) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT

Information on patent family members

International application No.

PCT/JP2021/016831

| Patent Documents referred in the Report | Publication Date | Patent Family | Publication Date |
|---|---|---|---|
| JP 2008-247778 A | 16 Oct. 2008 | (Family: none) | |
| JP 2020-011228 A | 23 Jan. 2020 | (Family: none) | |
| CN 102145287 A | 10 Aug. 2011 | (Family: none) | |

Form PCT/ISA/210 (patent family annex) (January 2015)

# EP 4 331 722 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHENGSHENG YANG ; RENTAO MU ; GUISHUO WANG ; JIMIN SONG ; HAO TIAN ; ZHI-JIAN ZHAO ; JINLONG GONG.** *Chemical Science,* 2019, vol. 10, 3161-3167

- **DI XU ; MINGYUE DING ; XINLIN HONG ; GUO-LIANG LIU ; SHIK CHI ; EDMAN TSANG.** *ACS Catalysis,* 2020, vol. 10, 5250-5260